# EUROPEAN PATENT SPECIFICATION

(11) **EP 0 163 491 B1**
(45) Date of publication and mention of the grant of the patent: **27.03.1996**
(21) Application number: 85303625.9
(22) Date of filing: 22.05.1985
(51) Int. Cl.: C12N 15/81, C12N 15/90, C12N 15/52, C12N 9/34

(54) **Yeast vector**
Hefevektor
Vecteur de levure

(30) Priority: 22.05.1984 US 612796
(43) Date of publication of application: 04.12.1985
(60) Divisional application: 95114460.9
(73) Proprietor: OMNIGENE INC, Cambridge Massachusetts 02139-9002 (US)
(72) Inventor: Yocum, Robert Rogers, Arlington, MA (US)
(74) Representative: Deans, Michael John Percy

(56) References cited:
- GENE, vol. 22, 1983; L. NAUMOVSKI et al., pp. 203-209
- NATURE, vol. 287, no. 5785, 30 October 1980, London/New York; A. JIMENEZ et al., pp. 869-871
- GENE, vol. 26, no. 1, December 1983, Amsterdam (NL); T.D. WEBSTER et al., pp. 243-252

## Description

This invention relates to genetic engineering in industrial yeast strains (i.e. non-haploid yeast cells).

Technology currently exists for introducing heterologous (i.e., modified or foreign) genes into laboratory strains of yeast of the genus Saccharomyces, particularly S. cerevisiae. Two types of plasmid vectors have been used for this purpose, replicating and integrating. Replicating vectors contain an origin of DNA replication that functions in yeast, so that the plasmid is maintained extrachromosomally, as a circular episome. Integrating vectors do not contain such an origin and therefore require insertion into a yeast chromosome to be stably maintained.

Both types of plasmids can be introduced into yeast cells by standard transformation methods. Since successful uptake and establishment of plasmid DNA by competent yeast cells is a relatively rare event (< 10⁻³), a selection mechanism is required to allow identification of transformants.

Most commonly, selection is accomplished by introducing auxotrophic mutations into the recipient yeast strain. The commonly used mutations are ura3, leu2, trp1, and his3. The plasmid of interest bears a wild type copy of one of these genes. Since the wild type copy on the plasmid is dominant to the host chromosomal allele, selection for cells that receive the plasmid is easily accomplished on a minimal medium lacking the nutrient that is required by the auxotrophic host cell.

There have also been reports of the use of antibiotic resistance to select transformed cells. Replicating vectors have been described that are based on the sensitivity of most Saccharomyces strains to the commercially available neomycin analog, antibiotic G418; Jiminez et al. (1980) Nature 287, 869; Hollenberg (1982) in Current Topics in Microbiology and Immunology, Hofschneider et al., eds. (Springer-Verlag, NY); Webster et al. (1983) Gene 26, 243. Webster et al. also describe an integrating plasmid vector which could not be directly selected for by resistance to G418. These vectors contain a gene, called kan^{r}, neo^{r}, or G418^{r} from the bacterial transposon Tn903, and a yeast origin of replication; the bacterial gene is preceded by its native bacterial promoter.

Naumovski and Friedberg "Construction of plasmid vectors that facilitate subcloning and recovery of yeast and E.coli DNA fragments" Gene 22 (1983) 203-209 suggested selecting a replicating plasmid for G418 resistance but gave no details. They describe a plasmid pNF2 which combines a gene encoding kanamycin-resistance, a 2µ origin of replication, and a sequence homologous to a yeast chromosomal sequence (URA3). They also describe the production of a plasmid pNF3 from pNF2 by deleting the 2µ replicating fragment. It is suggested that the resulting plasmid is integrating but no results are given.

Another replicating vector has been described which contains the gene for resistance to the antibiotic hygromycin B under the control of a yeast promoter; Gritz et al. (1983) Gene 25, 178.

The work described in all the above cases was concerned with haploid laboratory strains of yeast, and therefore has no direct relevance to industrial yeast strains which are not haploid.

In one aspect thereof, the invention features a yeast cell transformed by integration into a chromosome thereof of vector DNA; characterised in that the host yeast cell is an industrial non-haploid yeast cell; in that the vector DNA comprises a gene for resistance to an antibiotic otherwise capable of killing said yeast cell, said gene being transcribed from a promoter sequence which is capable of promoting the expression of said antibiotic resistance gene at a level which confers antibiotic resistance to said cell; in that said vector DNA comprises a sequence homologous with a sequence of said chromosome and is integrated therein; and in that said vector DNA further comprises a gene for a desired heterologous protein.

The second gene is a gene for a desired heterologous protein, and may be a non-yeast gene, a modified gene, a gene from a different yeast strain or an homologous gene from a different chromosomal location, but in each case encoding the desired protein.

Inclusion in the vector of a sequence which is homologous with a sequence (a "target" sequence) of the host chromosome facilitates integration. Preferably, the homologous sequence is separate from the control sequence which controls the antibiotic resistance gene.

In other preferred embodiments, the second gene encodes glucoamylase (which enables the generation of glucose from starch by the yeast cell), and the host cell participates in a process, e.g., the production of dough, which employs a product of the metabolism of the cell e.g. carbon dioxide.

In other preferred embodiments, the antibiotic resistance gene and the second gene are under the control of different promoters, the promoter controlling the second gene preferably being the more highly expressed of the two. The preferred integration method is one which results in the depositing of the heterologous gene in the host yeast cell chromosome, to the exclusion of much of the remainder of the vector DNA, providing greatly increased stability. Exclusion of this DNA also eliminates a potential source of interference with a characteristic, e.g., flavour, of the end product. Thus having selected transformants on the basis of antibiotic resistance, DNA unnecessary in the final product industrial yeast strain for its industrial purpose, and including for example the gene for antibiotic resistance, may be jettisoned.

Accordingly the invention provides in a second and alternative aspect thereof, an industrial non-haploid yeast cell including a chromosome comprising DNA of vector origin, said cell comprising a host yeast cell into the chromosome of which said DNA of vector origin has been integrated, or a descendant of such a host yeast cell into which said DNA of vector origin had been integrated; said DNA of vector origin comprising firstly a gene for a desired heterologous protein, and secondly a DNA sequence homologous with a sequence of said chromosome in said host yeast cell.

Integration of the vector into the host yeast cell chromosome provides stability over generations of host divisions in the absence of selection, an important advantage in industrial fermentation processes; replicating vectors can be lost from yeast cells at rates up to 1% to 5% per generation. Stable maintenance of integrated sequences over generations obviates the addition of toxic antibiotics to the fermentation medium to exert selective pressure to maintain the sequences. Our vectors also function well in yeast, by virtue of the yeast promoter sequence controlling the gene for antibiotic resistance.

For diploid or polyploid industrial yeast strains (as opposed to haploid laboratory strains), introduction into them of auxotrophic mutations (used for selection of transformants in haploid strains) is difficult. The use of antibiotic resistance in an integrating vector permits selection of stable transformants in non-haploid industrial yeast strains, regardless of number of chromosomes or the presence or absence of specific mutations.

Introduction of genes encoding heterologous enzymes into industrial yeast strains using our vectors will facilitate the production of such products as alcohol, which ordinarily relies on sugars to feed the yeast. An enzyme such as glucoamylase will enable the yeast to break down starch from inexpensive sources such as tapioca and potatoes to yield glucose, which can be fed on by the yeast. Similarly, bread-making can be made cheaper when starch (flour) rather than sugar is used as the primary energy source.

Heterologous enzymes can also facilitate the production of light beer, which has a lower starch content than regular beer. One method currently used in light beer brewing to break down residual starch which remains after completion of the malting process is to add to the wort glucoamylase derived from bread mould, a step which can be eliminated where the yeast used in brewing also carries and expresses a glucoamylase encoding gene.

Other enzymes can facilitate commercial fermentation processes in other respects. For example, in wine-making, insertion of the gene for malolactic enzyme or malate permease will permit host yeast cells to convert metabolized malic acid from grapes, thus inhibiting spoilage of the wine by removing malic acid, which is otherwise fed on by spoilage bacteria.

Other features and advantages of the invention will be apparent from the following description of the preferred embodiments, by way of example only.

We first briefly describe the drawings.

Figure 1 is a diagrammatic representation of a replicating vector.

Figures 2, 3a, and 3b are diagrammatic representations of integrating vectors.

Figure 4 is a diagrammatic representation of a mechanism by which an integrating vector is integrated into a host yeast chromosome.

### Plasmid Structure

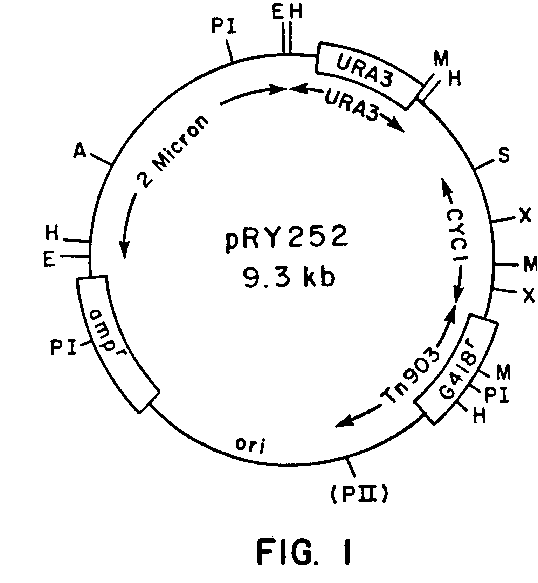

In Figs. 1-3, the following abbreviations are used for restriction endonuclease cleavage sites: A, Xbal; B, BamHI; E, EcoRI; H, HindIII; K, KpnI; M, SmaI; PI, PvuI; PII, PvuII; S, SalI; TII, SstII; U, Stul; X, Xhol. (A) denotes the position of a former Xbal site located about 3 kilobases away from the 5' end of the HO gene. This site was destroyed and replaced by a SalI site in the construction of pRY253. (PII) denotes a former PvuII site similarly destroyed during plasmid construction. Complete genes or gene fusions are shown by boxes. The abbreviations for the genes are as follows: amp^{r}, ampicillin resistance; G418^{r} antibiotic G418 resistance; HO, homothallism; CYCl, iso-1-cytochrome c; URA3, orotidine-5'-monophosphate decarboxylase; GAL1 galactokinase; lacZ, beta-galactosidase. The concentric arrows inside the circles indicate segments of DNA whose origin is other than pBR322. The extent of these sequences is indicated by the arrowheads and the source is indicated by the labels. pBR322 sequences have no concentric arrows. Other abbreviations are: kb, kilobase pairs; ori, E. coli origin of replication.

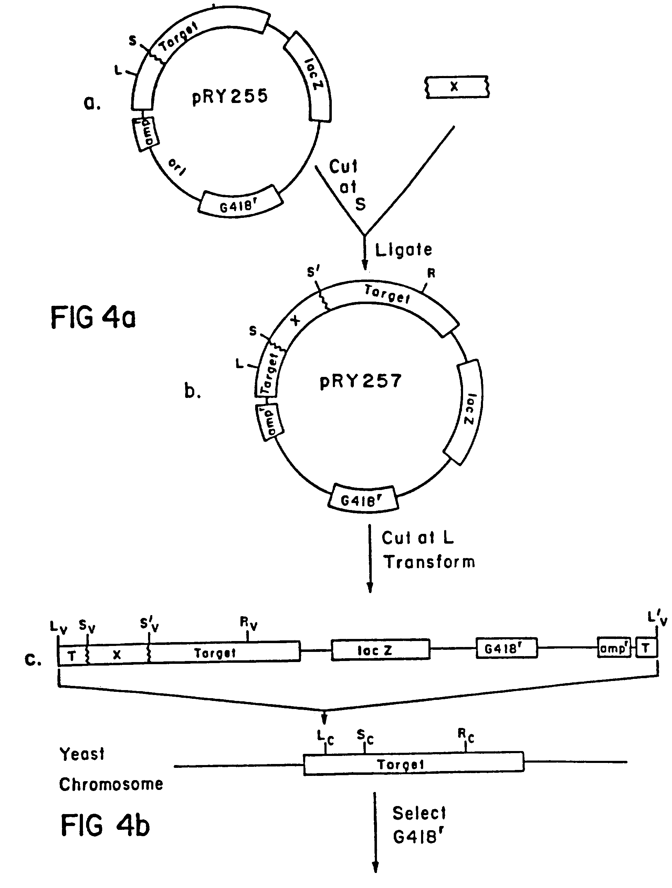

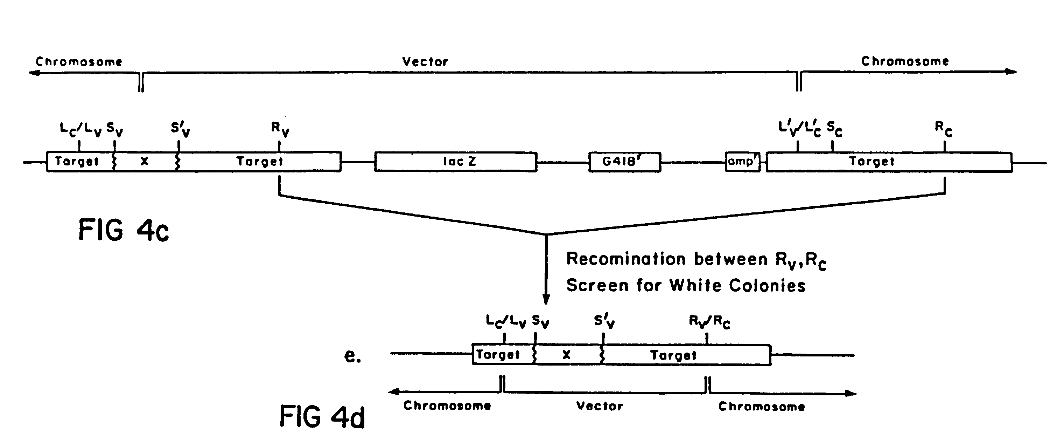

In Fig. 4, the following abbreviations are used: X, any gene or DNA sequence to be integrated into a yeast chromosome and expressed; S, a cloning site (for example: a restriction endonuclease site) into which gene X is inserted; L, a site (for example, a restriction endonuclease site) for linearizing the vector within the target sequence, "T" or "Target"; R, a site, not necessarily specific, where recombination between homologous vector-derived and chromosome-derived target sequences occurs. An apostrophe designates half of a site (such as S or L) that is separated from its other half by cleavage, by insertion of an intervening DNA sequence, or by integration into a chromosome. A subscript of V or C designates sites or portions of sites that are derived from the vector or chromosome, respectively. Other abbreviations are as in Fig. 1-3.

Referring to Fig. 1, replicating plasmid vector pRY252 is composed, beginning at the 12 o'clock position of the drawing and moving clockwise, of sequence E-H, a small piece of DNA from the E. coli plasmid pBR322; sequence H-H, which includes the yeast URA3 gene (one of the genes required for the ability to grow on uracil-deficient media; this gene is an unnecessary artifact in the plasmid which was originally inserted to provide a comparative selection means); sequence H-S, another piece of pBR322; sequence S-(PII), which includes the yeast CYCl (cytochrome c) promoter and most of the gene for resistance to G418 from the bacterial transposon Tn903 (the non-essential N-terminal region is not included); sequence (PII)-E, including the E. coli origin of replication from pBR322 and the amp^{r} gene for selecting transformants in E. coli; and sequence E-E, the yeast origin of replication from a yeast 2 micron circle.

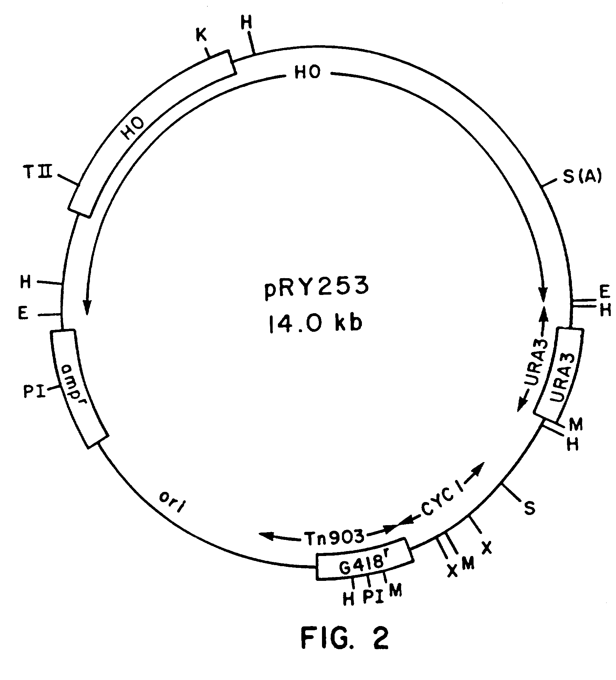

Referring to Figure 2, integrating plasmid vector pRY253 is derived from pRY252 in that the yeast origin of replication sequence is replaced by a 7.0 kb EcoRI fragment of S. cerevisiae containing the HO (homothallism) gene, including site K for insertion of a desired heterologous gene.

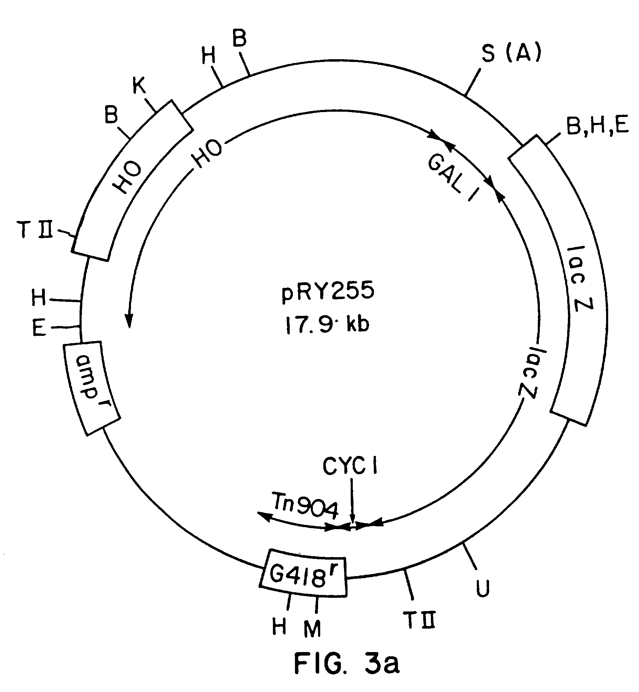

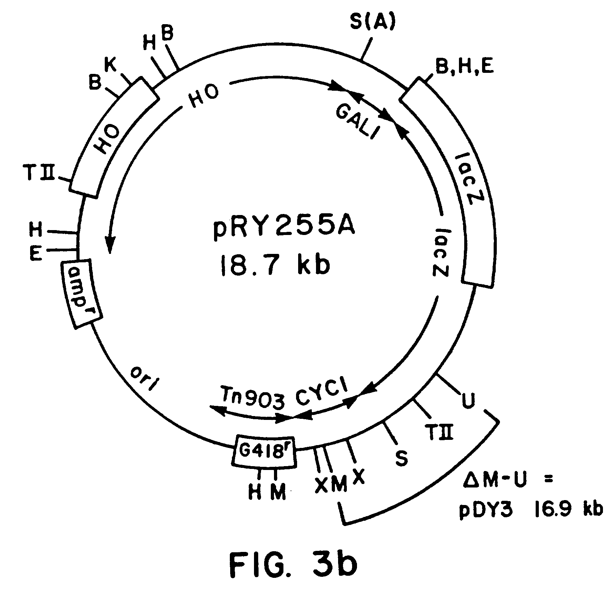

Referring to Fig. 3a, integrating plasmid vector pRY255 is derived from pRY253 in that a 3.3 kilobase SalI to XhoI fragment extending from one end of the HO insert to the beginning of the CYCI promoter sequence and containing the URA3 gene has been replaced with a 6.0 kilobase Xhol to SalI fragment containing a gene fusion of the yeast GALl gene and the E. coli lacZ gene. Referring to Fig. 3b, pRY255A is similar to pRY255, in that it is also derived from pRY253, in that the 6.0 kilobase Xhol to SalI fragment containing the GALl-lacZ fusion is substituted for the 2.5 kilobase SalI fragment of pRY253.

Referring also to Figure 3b, pDY3 was derived from pRY255A by deleting the 1.8 kilobase region between a Stul site and the Smal site upstream from the CYCl promoter.

pRY255A and pDY3 can be used in substantially the same way as pRY255, as described below. In addition, in pDY3, an SstII site near the 3' end of the HO is unique on the vector, making it a more convenient site for linearization of the vector.

Plasmids pRY252, pRY253, pRY255, and pRY255A were deposited in the American Type Culture Collection, Rockville, Maryland, and have the depository numbers, respectively, ATCC 39687, 39688, 39689, and 39822.

Referring to Fig. 4, plasmid pRY257 will contain a gene X for a desired heterologous protein, e.g., glucoamylase or interferon, inserted at site S in plasmid pRY255.

### Plasmid Construction

The plasmids illustrated in Figs. 1-3 were made using conventional recombinant DNA methods and publicly available materials.

Plasmids pRY253, pRY255, pRY255A, and pDY3 were derived from replicating plasmid pRY252 which, briefly, was constructed as follows.

The URA3 gene was inserted into plasmid pBR322 as illustrated, and then the origin of replication from the endogenous yeast 2 micron circle, without the three genes normally accompanying it, was inserted. The vector is able to replicate in host yeast cells without containing these three genes, two of which encode proteins essential for replication, because host yeast cells already contain the endogenous 2 micron circle encoding those proteins (Botstein et al. (1979) Gene 8, 17).

The CYCl-G418^{r} fusion portion of the plasmid was constructed by fusing the XhoI site near the 5' end of the G418^{r} gene of transposon Tn903 (described in Oka et al. (1981) J. Mol. Biol. 147, 217) to the BamHI site following the CYCl promoter and the 5' end of the CYCl coding sequences of plasmid pLG669 (described in Guarente et al. (1981) PNAS USA 78, 2199) after rendering both ends flush with mung bean nuclease. The DNA sequence of this fusion junction is (CYC1) . . . TAAATTAATAATGACCG⇓GGCCG . . . (G418^{r}). The arrow shows the point of fusion.

Plasmids pRY253, pRY255, pRY255A, and pDY3 were constructed from pRY252 by making the gene fragment substitutions and deletions shown in the Figures. Plasmid pRY257 can be constructed by inserting a gene X for a desired protein at site S of pRY255, within the HO gene, so that there are portions of the HO gene on either side of gene X, as shown in Fig. 4.

### Plasmid Use

Our vectors can be used in any useful process in which host cells of an industrial yeast strain express a gene for a desired heterologous protein. The desired heterologous protein gene can be inserted using conventional recombinant DNA techniques, e.g., as described in Maniatis et al. (1982) Molecular Cloning: A Laboratory Manual Cold Spring Harbor Press, Cold Spring Harbor, New York, the disclosure of which is to be regarded as hereby incorporated by reference. pRY253, pRY255, pRY255A, pDY3 can also be used to delete genes from wild type yeast strains. For the deletion of genes, the HO portion of the vectors becomes irrelevant. Deletion of a gene or a portion of a gene can be accomplished as follows:
1. Clone the gene to be deleted with some adjacent sequence on both sides of the gene.
2. Create a deletion of the cloned gene in vitro, leaving a portion of each of the 3' and 5' ends of the gene sufficient for homologous recombination but insufficient to encode the protein normally encoded by the gene.
3. Place the deletion-containing DNA at an appropriate location in one of the integration vectors described herein.
4. Linearize the vector at a point in one of the sequences adjacent to the deletion, and perform integration transformation, selecting for G418 resistance.
5. Grow a stable transformant for 20 to 40 generations non-selectively, and screen for vector jettisoning events by either loss of blue colony color on Xgal indicator plates or by replica plating to G418 containing plates.
6. Screen among colonies that have jettisoned the vector for those that retained the deleted version of the gene by Southern blotting.

The vectors are particularly useful in industrial yeast strains used in the production of end products such as wine, bread, and beer which involve carbohydrate fermentation.

### Transformation

Yeast cells were transformed with vectors as follows.

Laboratory yeast strain DBY 745 (described in Guarente et al. (1981) PNAS USA 78, 2199); a Carlsberg® brewing strain (isolated from unpasteurized beer); Fleischman's® baking yeast (purchased at a supermarket); and a Bordeaux wine yeast (ATCC 42928) were grown in a standard rich medium, YEP-D, spheroplasted with glusulase, and exposed to plasmid DNA by standard methods of yeast transformation, as described in Sherman et al. (1981) Methods in Yeast Genetics (Cold Spring Harbor Laboratories Press, Cold Spring Harbor, New York). Integrating plasmid pRY253 was linearized by restriction endonuclease digestion, at a unique SstII site near the 3' end of the HO gene, and pRY255 was linearized at a unique KpnI site near the 5' end of the HO gene, prior to transformation, in order to direct integration at the HO locus. Replicating plasmid pRY252 was not linearized.

After exposure to the plasmids, 10⁸ spheroplasts were grown in YEP-D plus 1.0 M sorbitol for 30 minutes at 30°C and then plated in 6 ml of warm 3% agarose containing YEP-D over 20 ml of 2% agar. YEP-D, 1.0 M sorbitol, and 70 mM potassium phosphate, pH 7.0. After 10 minutes of cooling at room temperature, another 4 ml of warm 1% agar, YEP-D, 1.0 M sorbitol was layered over the top agar. The cells were then allowed to grow at 30°C for 6 generations, corresponding to 8 hours for DBY 745, 9 hours for Carlsberg, 7 hours for the wine yeast, and 6 hours for Fleishman's. After this "growing out" period, 0.6 ml of a sterile solution of antibiotic G418 at 25 mg/ml was spread over the agar surface and allowed to dry in a sterile hood. The plates were then incubated for 2-5 days at 30°C, after which time colonies appeared out of a background of untransformed cells. Several of these colonies were toothpicked onto fresh YEP-D plates containing 500 µg/ml G418. Cells that were successfully transformed gave rise to visible colonies within 24 hours, while untransformed cells did not. A summary of these results is given in Table 1, below.

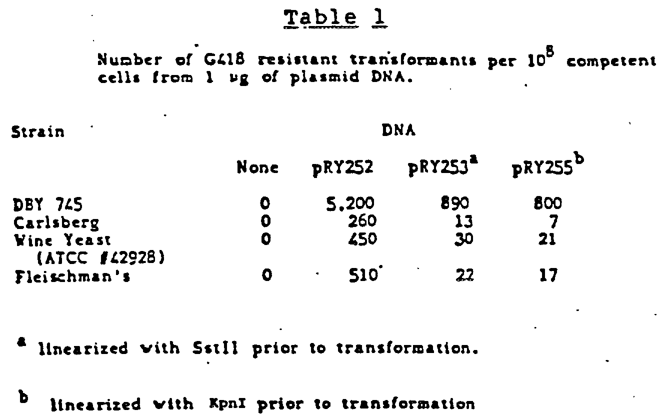

Transformants obtained from the integrating plasmids pRY253 and pRY255 were shown to contain stably integrated plasmids, by growing isolated transformants for 10 to 20 generations non-selectively in YEP-D and 5 showing that reversion to G418 sensitivity occurred at a rate less than 10⁻³. Transformants containing pRY255 gave blue colonies on plates containing galactose as the sole carbon source, 70 mM potassium phosphase buffer, pH 7.0, and Xgal indicator dye (5'-bromo-4'-chloro-3'-indoyl-beta-D-galactoside). Jettisoning of Vector Sequences

Plasmid vector pRY257 can be linearized and used to transform host cells of industrial yeast strains , as described above for plasmids pRY253 and pRY255. As described above, transformants are selected on the basis of antibiotic resistance (Fig. 4).

Following this selection, as shown in Fig. 4, a further step can be taken to jettison unnecessary portions of the vector which might adversely affect transformant stability, adversely affect the taste or any other important property of the end product, or waste metabolic energy. In effect, this screening step "deposits" the desired gene in the host chromosome, while excluding extraneous DNA. The exclusion of this extraneous DNA increases transformant stability by eliminating tandem repeat sequences which could cause undesirable recombination events resulting, for example, in loss of the desired heterologous gene. Also, elimination of the gene for antibiotic resistance can be an advantage if for some reason it is anticipated that the use of the antibiotic to kill the yeast may become necessary. Finally, elimination of all Escherichia coli derived sequences from the transformed yeast may simplify governmental regulatory clearance for use of the organism.

The screening depends on the presence in the vector of a gene encoding a screenable trait; in pRY257, this is the E. coli lacZ gene which encodes beta-galactosidase. Yeast colonies that express this gene turn blue on an appropriate indicator petri plate containing a colorimetric indicator dye such as Xgal. Thus to select transformants in which a portion of the vector DNA, including the lacZ gene, has been jettisoned. transformants are plated onto an indicator plate, and those colonies remaining white on the plates selected as the transformants, or descendants of transformants, not retaining the lacZ gene.

Fig. 4 illustrates the jettisoning mechanism. In some transformants there will be a cross-over event between vector sequences homologous with chromosome sequences (see Fig. 4d). This cross-over event causes the looping out and deletion of the region of the vector between the homologous sequences (see Fig. 4e). If desired heterologous gene X (encoding, say, glucoamylase) is outside this region, it remains deposited in the chromosome. The frequency of this type of event can be increased relative to that of other unwanted events (such as looping out of the entire plasmid including the deposited gene) by placing the deposited gene nearer to the end of the target sequences containing the linearization site than to the end of the target sequences that contain the "looping out" site.

The desired looping out event can be distinguished from undesired events by screening among white colonies for those that maintain gene X. This can be done either by a functional assay for the product of gene X (e.g., in the case of glucoamylase, halos on starch-containing plates), or by direct assay for the presence of gene X by Southern blotting techniques. The two screenings can be carried out at once, e.g. by using a medium containing both Xgal and starch.

Other embodiments are within the teaching of the invention.

For example, although the frequency at which the integrating vectors integrate into the host chromosome is increased by linearizing the vector prior to transformation, integration, at a lower frequency, can be achieved by transformation with the vectors in circularized form. Although the HO gene is the most preferred target gene, any other region of the lost chromosome not involved in metabolism can be used.

In addition to enzymes involved in the production of bread and alcoholic beverages, the vectors can be used in processes in which the desired end product is the protein, e.g., therapeutic proteins such as interferon, encoded by the inserted heterologous gene. The heterologous gene can also be a gene already carried on a different portion of the host chromosome; for example, it might be advantageous to add an additional copy of a native gene involved in alcohol production, to increase production levels.

The promoter sequence controlling the gene for antibiotic resistance can also vary widely, the only crucial factor being that the sequence provides that a sufficient level of expression in yeast cells is maintained.

When a gene in the host chromosome is targeted by employing a vector containing a homologous sequence, linearization of the vector prior to transformation can occur anywhere within the homologous sequence; generally, however, integration efficiency is improved when linearization occurs near the center of the sequence, and decreases as the linearizaton point approaches either end of the sequence.

The screenable trait, in addition to the ability to produce beta-galactosidase, can be any trait whose absence can be detected. In addition, when beta-galactosidase production is used, the gene need not be the E. coli lacZ gene; for example. the LAC4 gene from Kluyeromyces species, e.g., K. lactis, which also encodes a beta-galactosidase, can also be used.

## Claims

1. A yeast cell transformed by integration into a chromosome thereof of vector DNA; characterised in that the host yeast cell is an industrial non-haploid yeast cell; in that the vector DNA comprises a gene for resistance to an antibiotic otherwise capable of killing said yeast cell, said gene being transcribed from a promoter sequence which is capable of promoting the expression of said antibiotic resistance gene at a level which confers antibiotic resistance to said cell; in that said vector DNA comprises a sequence homologous with a sequence of said chromosome and is integrated therein; and in that said vector DNA further comprises a gene for a desired heterologous protein.

2. A cell according to Claim 1, said vector DNA being further characterised in that said gene for a desired heterologous protein is transcribed from a promoter sequence different from the promoter sequence from which said gene for antibiotic resistance is transcribed.

3. A cell according to Claim 2, said vector DNA being further characterised in that said gene for a desired heterologous protein is more highly expressed by its promoter sequence than said antibiotic resistance gene is expressed by its promoter sequence.

4. A cell according to Claim 1, said vector DNA being further characterised in that said homologous sequence of said vector does not comprise a portion of said promoter sequence.

5. A cell according to any of Claims 1 to 4, further characterised in that said sequence of said chromosome comprises at least a portion of said host yeast cell's gene for homothallism.

6. A cell according to Claim 1, wherein the chromosomal DNA of said host yeast cell comprises a target, said target comprising, in the following order:
(a) a first end of said target,
(b) a first target sequence bounded by said first end and by a site Lc,
(c) said site Lc,
(d) a second target sequence bounded by said site Lc and by a site Sc,
(e) said site Sc,
(f) a third target sequence bounded by a site Sc and by the second end of said target, and
(g) said second end of said target;
said vector being further characterised in that it is linearized; in that it comprises the following sequences:
(i) a first vector sequence homologous with said second target sequence,
(ii) a second vector sequence homologous with said first target sequence,
(iii) a third vector sequence homologous with said third target sequence,
(iv) said gene for a desired heterologous protein, and
(v) said antibiotic resistance gene;
and in that said first vector sequence and said second vector sequence are separated from each other by a region of partial non-homology which comprises said sequences (iii), (iv), and (v).

7. A cell according to Claim 6, said vector DNA being further characterised in comprising a sequence (vi), a third gene for a screenable trait, and in that said region of partial non-homology comprises said sequences (iii), (iv), (v) and (vi).

8. A cell according to Claim 7, said vector being further characterised in that said sequences are arranged on said linearized vector in the order (i), (iv), (iii), (vi), (v), (ii).

9. A cell according to Claims 7 or 8, said vector being further characterised in that said third gene encodes beta-galactosidase.

10. A cell according to any preceding claim, said vector being further characterised in that said gene for antibiotic resistance comprises a gene for resistance to G418.

11. An industrial non-haploid yeast cell including a chromosome comprising DNA of vector origin, said cell comprising a host yeast cell into the chromosome of which said DNA of vector origin has been integrated, or a descendant of such a host yeast cell into which said DNA of vector origin had been integrated; said DNA of vector origin comprising firstly a gene for a desired heterologous protein, and secondly a DNA sequence homologous with a sequence of said chromosome in said host yeast cell.

12. A cell according to any preceding claim, further characterised in that said gene for a desired heterologous protein encodes glucoamylase.

13. A cell according to any of Claims 1 to 11, further characterised in that said gene for a desired heterologous protein encodes malolactic enzyme or malate permease.

14. A cell according to Claim 12, further characterised in that said vector enables the expression of glucoamylase in said host yeast cell.

15. A cell according to Claims 12 or 14, characterised in that said glucoamylase enables said cell to generate glucose from starch.

16. A cell according to Claim 15, further characterised in that said cell participates in a process employing a product of the metabolism of said cell, said cell employing said glucose as an energy source in said process.

17. A cell according to Claim 16, further characterised in that said product of metabolism is carbon dioxide and said process is the production of dough.

18. A cell according to Claim 16, further characterised in that said product of metabolism is ethanol.

19. A cell according to Claim 13, further characterised in that said vector DNA enables the expression of said malolactic enzyme or malate permease in said host yeast cell.

20. A cell according to Claims 13 or 19, characterised in that said malolactic enzyme or malate permease enables said cell to metabolize malic acid.

21. A method for directing integration of vector DNA into the chromosome of an industrial non-haploid yeast cell by use of a cloning vector comprising the vector DNA specified in any of Claims 1 or 6 to 9, and the method comprising linearizing said vector in said homologous sequence and then exposing said linearized vector to said host yeast cell under transforming conditions.

22. A method for directing the integration into the chromosomal DNA of an industrial non-haploid yeast strain as defined in Claim 6 of a linearized vector as defined in Claim 6, whereby at least a portion of said region of partial non-homology is jettisoned from said vector, said method comprising
exposing said linearized vector to said host yeast cells under transforming conditions,
selecting as transformants yeast cells exhibiting resistance to said antibiotic, and
isolating a subgroup of said transformants which no longer exhibit resistance to said antibiotic.

23. A method for directing integration of vector DNA into the chromosomal DNA of an industrial non-haploid yeast strain as defined in any of Claims 7, 8 or 9 by use of a linearized vector as defined in any of Claims 7, 8 or 9 respectively, whereby at least a portion of said region of partial non-homology is jettisoned from said vector, said method comprising
exposing said linearized vector to said host yeast cells under transforming conditions,
isolating as transformants yeast cells exhibiting resistance to said antibiotic, and
isolating a subgroup of said transformants or descendants of said transformants not exhibiting said screenable trait.

24. The method of Claim 23, employing the vector defined in Claim 9, wherein isolating of said subgroup is carried out by growing said transformants on medium which changes colour in the presence of beta-galactosidase and then selecting transformants not causing said colour change.

25. The vector pRY253 (ATCC No. 39688).

26. The vector pRY255 (ATCC No. 39689).

27. The vector pRY255A (ATCC No. 39822).

28. A method of deleting a gene from the chromosome of an industrial non-haploid yeast cell comprising
transforming said yeast cell with a vector comprising a gene for resistance to an antibiotic otherwise capable of killing said yeast cell, said gene being transcribed from a promoter sequence which is capable of promoting the expression of said antibiotic resistance gene at a level which confers antibiotic resistance to said cell; said vector comprising a sequence homologous with a sequence of said chromosome, and further DNA corresponding to the gene to be deleted, said DNA comprising a DNA sequence having 3' and 5' ends homologous with the 3' and 5' ends of said gene to be deleted and lacking the complete gene to be deleted, and
isolating transformants lacking said gene to be deleted.

29. Use of cells according to Claim 11 to produce ethanol.

30. Use of cells according to Claim 11 to brew light beer.

31. Use of cells according to Claim 11 in the production of dough.

32. Use of cells according to Claim 11 in the baking of bread products.

33. Use of cells according to Claim 11 in the fermentation of wine.

## Patentansprüche

1. Hefezelle, welche durch Integration einer Vektor-DNA in eines ihrer Chromosomen transformiert ist, dadurch gekennzeichnet, daß
die Hefe-Wirtszelle eine industrielle, nicht-haploide Hefezelle ist,
die Vektor-DNA ein Resistenzgen gegen ein Antibiotikum enthält, das andernfalls die Hefezelle töten kann, wobei das Gen ausgehend von einer Promotor-Sequenz transkribiert wird, die die Expression des Antibiotikum-Resistenzgens auf ein Niveau steigern kann, das der Zelle Antibiotikum-Resistenz verleiht,
die Vektor-DNA eine Sequenz umfaßt, die zu einer Sequenz des Chromosoms homolog ist und darin integriert wird, und daß
die Vektor-DNA weiter ein Gen für ein gewünschtes heterologes Protein umfaßt.

2. Zelle nach Anspruch 1, worin die Vektor-DNA weiter dadurch gekennzeichnet ist, daß das Gen für ein gewünschtes heterologes Protein ausgehend von einer Promotor-Sequenz transkribiert wird, die von der Promotor-Sequenz, von der ausgehend das Antibiotikum-Resistenzgen transkribiert wird, verschieden ist.

3. Zelle nach Anspruch 2, worin die Vektor-DNA weiter dadurch gekennzeichnet ist, daß das Gen für ein gewünschtes heterologes Protein durch dessen Promotor-Sequenz stärker exprimiert wird, als das Antibiotikum-Resistenzgen durch dessen Promotor-Sequenz.

4. Zelle nach Anspruch 1, worin die Vektor-DNA weiter dadurch gekennzeichnet ist, daß die homologe Sequenz des Vektors keinen Teil der Promotor-Sequenz umfaßt.

5. Zelle nach einem der Ansprüche 1 bis 4, weiter dadurch gekennzeichnet, daß die Sequenz des Chromosoms mindestens einen Teil des Gens für Homothallismus der Hefe-Wirtszelle umfaßt.

6. Zelle nach Anspruch 1, worin die chromosomale DNA der Hefe-Wirtszelle ein Ziel enthält, das in der folgenden Reihenfolge umfaßt:
(a) ein erstes Ende des Ziels
(b) eine erste Zielsequenz, die durch das erste Ende und durch eine Stelle Lc begrenzt ist,
(c) die Stelle Lc,
(d) eine zweite Zielsequenz, die durch die Stelle Lc und eine Stelle Sc begrenzt ist,
(e) die Stelle Sc,
(f) eine dritte Zielsequenz, die durch eine Stelle Sc und durch das zweite Ende des Ziels begrenzt ist, und
(g) das zweite Ende des Ziels,
worin der Vektor weiter dadurch gekennzeichnet ist, daß er linearisiert ist und die folgenden Sequenzen enthält:
(i) eine erste Vektor-Sequenz, die zu der zweiten Zielsequenz homolog ist,
(ii) eine zweite Vektor-Sequenz, die zu der ersten Zielsequenz homolog ist,
(iii) eine dritte Vektor-Sequenz, die zu der dritten Zielsequenz homolog ist,
(iv) das Gen für ein gewünschtes heterologes Protein, und
(v) das Antibiotikum-Resistenzgen, und
daß die erste Vektor-Sequenz und die zweite Vektor-Sequenz durch einen teilweise nicht-homologen Bereich voneinander getrennt sind, der die Sequenzen (iii), (iv) und (v) enthält.

7. Zelle nach Anspruch 6, worin die Vektor-DNA weiter dadurch gekennzeichnet ist, daß sie eine Sequenz (vi), ein drittes Gen zum Screenen nach einem Merkmal enthält, und daß der teilweise nicht-homologe Bereich die Sequenzen (iii), (iv), (v) und (vi) enthält.

8. Zelle nach Anspruch 7, worin der Vektor weiter dadurch gekennzeichnet ist, daß die Sequenzen auf dem linearisierten Vektor in der Reihenfolge (i), (iv), (iii), (vi), (v), (ii) angeordnet sind.

9. Zelle nach Anspruch 7 oder 8, worin der Vektor weiter dadurch gekennzeichnet ist, daß das dritte Gen β-Galactosidase codiert.

10. Zelle nach einem der vorhergehenden Ansprüche, worin der Vektor weiter dadurch gekennzeichnet ist, daß das Antibiotikum-Resistenzgen ein Gen zur Resistenz gegenüber G418 enthält.

11. Industrielle, nicht-haploide Hefezelle, welche ein Chromosom beinhaltet, das eine von einem Vektor stammende DNA enthält, wobei die Zelle eine Hefe-Wirtszelle umfaßt, in deren Chromosom die von einem Vektor stammende DNA integriert wurde, oder einen Abkömmling einer derartigen Hefe-Wirtszelle, in den die von einem Vektor stammende DNA integriert wurde, wobei die von einem Vektor stammende DNA erstens ein Gen für ein gewünschtes heterologes Protein und zweitens eine DNA-Sequenz enthält, die zu einer Sequenz des Chromosoms in der Hefe-Wirtszelle homolog ist.

12. Zelle nach einem der vorhergehenden Ansprüche, weiter dadurch gekennzeichnet, daß das Gen für ein gewünschtes heterologes Protein Glucoamylase codiert.

13. Zelle nach einem der Ansprüche 1 bis 11, weiter dadurch gekennzeichnet, daß das Gen für ein gewünschtes heterologes Protein Malomilch-Enzym oder Malat-Permease codiert.

14. Zelle nach Anspruch 12, weiter dadurch gekennzeichnet, daß der Vektor Expression von Glucoamylase in der Hefe-Wirtszelle ermöglicht.

15. Zelle nach Anspruch 12 oder 14, weiter dadurch gekennzeichnet, daß die Glucoamylase die Zelle zur Herstellung von Glucose aus Stärke befähigt.

16. Zelle nach Anspruch 15, weiter dadurch gekennzeichnet, daß die Zelle an einem Verfahren beteiligt ist, in dem ein Metabolismus-Produkt der Zelle verwendet wird, wobei die Zelle in dem Verfahren die Glucose als eine Energiequelle verwendet.

17. Zelle nach Anspruch 16, weiter dadurch gekennzeichnet, daß das Metabolismus-Produkt Kohlendioxid ist und das Verfahren die Herstellung von Teig ist.

18. Zelle nach Anspruch 16, weiter dadurch gekennzeichnet, daß das Metabolismus-Produkt Ethanol ist.

19. Zelle nach Anspruch 13, weiter dadurch gekennzeichnet, daß die Vektor-DNA Expression des Malomilch-Enzyms oder der Malat-Permease in der Hefe-Wirtszelle ermöglicht.

20. Zelle nach Anspruch 13 oder 19, weiter dadurch gekennzeichnet, daß das Malomilch-Enzym oder die Malat-Permease die Zelle dazu befähigt, Äpfelsäure zu metabolisieren.

21. Verfahren zum Steuern der Integration von Vektor-DNA in das Chromosom einer industriellen, nicht-haploiden Hefezelle unter Verwendung eines Klonierungs-Vektors, der die Vektor-DNA nach einem der Ansprüche 1 oder 6 bis 9 enthält, wobei das Verfahren umfaßt, den Vektor in die homologe Sequenz zu linearisieren und den linearisierten Vektor dann der Hefe-Wirtszelle unter Transformations-Bedingungen auszusetzen.

22. Verfahren zum Steuern der Integration eines linearisierten Vektors nach Anspruch 6 in die chromosomale DNA eines industriellen, nicht-haploiden Hefe-Stamms nach Anspruch 6, wobei mindestens ein Teil des teilweise nicht-homologen Bereichs von dem Vektor abgetrennt wird, wobei das Verfahren umfaßt
den linearisierten Vektor den Hefe-Wirtszellen unter Transformations-Bedingungen auszusetzen,
als Transformanten Hefezellen auszuwählen, die Resistenz gegenüber dem Antibiotikum zeigen, und
eine Untergruppe der Transformanten zu isolieren, die keine Resistenz mehr gegenüber dem Antibiotikum zeigen.

23. Verfahren zum Steuern der Integration von Vektor-DNA in die chromosomale DNA eines industriellen, nicht-haploiden Hefe-Stamms nach einem der Ansprüche 7, 8 oder 9 unter Verwendung eines linearisierten Vektors nach einem der Ansprüche 7, 8 bzw. 9, wobei mindestens ein Teil des teilweise nicht-homologen Bereichs von dem Vektor entfernt wurde, wobei das Verfahren umfaßt
den linearisierten Vektor den Hefe-Wirtszellen unter Transformations-Bedingungen auszusetzen,
als Transformanten Hefezellen zu isolieren, die Resistenz gegenüber dem Antibiotikum zeigen, und
eine Untergruppe der Transformanten oder Abkömmlinge der Transformanten, die das screenbare Merkmal nicht zeigen, zu isolieren.

24. Verfahren nach Anspruch 23, wobei der Vektor nach Anspruch 10 verwendet wird, wobei die Isolierung der Untergruppe dadurch durchgeführt wird, daß die Transformanten auf einem Medium gezüchtet werden, das in Anwesenheit von β-Galactosidase die Farbe ändert und dann Transformanten, die diese Farbänderung nicht bewirken, ausgewählt werden.

25. Vektor pRY253 (ATCC Nr.39688).

26. Vektor pRY255 (ATCC Nr.39689).

27. Vektor pRY255A (ATCC Nr.39822).

28. Verfahren zur Entfernung eines Gens aus dem Chromosom einer industriellen, nicht-haploiden Hefezelle, welches umfaßt
Transformieren der Hefezelle mit einem Vektor, der ein Resistenzgen gegen ein Antibiotikum enthält, das andernfalls die Hefezelle abtöten kann, wobei das Gen ausgehend von einer Promotor-Sequenz transkribiert wird, die die Expression des Antibiotikum-Resistenzgens auf ein Niveau steigern kann, das der Zelle Antibiotikum-Resistenz verleiht, wobei der Vektor eine Sequenz enthält, die zu einer Sequenz des Chromosoms homolog ist, und weiter DNA enthält, die dem zu entfernenden Gen entspricht, wobei die DNA eine DNA-Sequenz umfaßt, die 3'-und 5'-Enden besitzt, die zu den 3'- und 5'-Enden des zu entfernenden Gens homolog sind und der das vollständige, zu entfernende Gen fehlt, und
Isolieren der Transformanten, denen das zu entfernende Gen fehlt.

29. Verwendung von Zellen nach Anspruch 11 zur Herstellung von Ethanol.

30. Verwendung von Zellen nach Anspruch 11 zum Brauen von kalorienarmen Bier.

31. Verwendung von Zellen nach Anspruch 11 bei der Herstellung von Teig.

32. Verwendung von Zellen nach Anspruch 11 beim Backen von Brotprodukten.

33. Verwendung von Zellen nach Anspruch 11 bei der Fermentation von Wein.

## Revendications

1. Cellule de levure transformée par intégration dans un de ses chromosomes d'un ADN de vecteur, caractérisée en ce que la cellule de levure hôte est une cellule de levure non haploïde industrielle, en ce que l'ADN de vecteur comprend un gène de résistance à un antibiotique sinon capable de tuer ladite cellule de levure, ledit gène étant transcrit à partir d'une séquence promoteur qui est capable de favoriser l'expression dudit gêne de résistance à l'antibiotique à un niveau qui confère une résistance à l'antibiotique à ladite cellule, en ce que ledit ADN de vecteur comprend une séquence homologue à une séquence dudit chromosome et y est intégré et en ce que ledit ADN de vecteur comprend de plus un gêne d'une protéine hétérologue souhaitée.

2. Cellule selon la revendication 1, ledit ADN de vecteur étant de plus caractérisé en ce que ledit gène d'une protéine hétérologue souhaitée est transcrit à partir d'une séquence promoteur différente de la séquence promoteur à partir de laquelle ledit gène de résistance à l'antibiotique est transcrit.

3. Cellule selon la revendication 2, ledit ADN de vecteur étant de plus caractérisé en ce que ledit gène d'une protéine hétérologue souhaitée est exprimé de façon plus importante par sa séquence promoteur que ledit gène de résistance à l'antibiotique n'est exprimé par sa séquence promoteur.

4. Cellule selon la revendication 1, ledit ADN de vecteur étant de plus caractérisé en ce que ladite séquence homologue dudit vecteur ne comprend pas une portion de ladite séquence promoteur.

5. Cellule selon l'une quelconque des revendications 1 à 4, caractérisée de plus en ce que ladite séquence dudit chromosome comprend au moins une portion du gène d'homothallie de ladite cellule de levure hôte.

6. Cellule selon la revendication 1, dans laquelle l'ADN chromosomique de ladite cellule de levure hôte comprend une cible, ladite cible comprenant dans l'ordre :
(a) une première extrémité de ladite cible,
(b) une première séquence de cible liée par ladite première extrémité et par un site Lc,
(c) ledit site Lc,
(d) une deuxième séquence de cible liée par ledit site Lc et par un site Sc,
(e) ledit site Sc,
(f) une troisième séquence de cible liée par le site Sc et par la deuxième extrémité de ladite cible et
(g) ladite deuxième extrémité de ladite cible ;
ledit vecteur étant de plus caractérisé en ce qu'il est linéarisé, en ce qu'il comprend les séquences suivantes :
(i) une première séquence de vecteur homologue à ladite deuxième séquence de cible,
(ii) une deuxième séquence de vecteur homologue à ladite première séquence de cible,
(iii) une troisième séquence de vecteur homologue à ladite troisième séquence de cible,
(iv) ledit gène d'une protéine hétérologue souhaitée et
(v) ledit gène de résistance à l'antibiotique,
et en ce que ladite première séquence de vecteur et ladite deuxième séquence de vecteur sont séparées l'une de l'autre par une région de non homologie partielle qui comprend lesdites séquences (iii), (iv) et (v).

7. Cellule selon la revendication 6, ledit ADN de vecteur étant de plus caractérisé en ce qu'il comprend une séquence (vi), à savoir un troisième gène d'un caractère pouvant être criblé, et en ce que ladite région de non homologie partielle comprend lesdites séquences (iii), (iv), (v) et (vi).

8. Cellule selon la revendication 7, ledit vecteur étant de plus caractérisé en ce que lesdites séquences sont placées sur ledit vecteur linéarisé dans l'ordre (i), (iv), (iii), (vi), (v), (ii).

9. Cellule selon les revendications 7 ou 8, edit vecteur étant de plus caractérisé en ce que le troisième gène code pour la β-galactosidase.

10. Cellule selon l'une quelconque des revendications précédentes, ledit vecteur étant de plus caractérisé en ce que ledit gène de résistance à un antibiotique comprend un gène de résistance à G418.

11. Cellule de levure non haploïde industrielle contenant un chromosome comprenant un ADN d'origine de vecteur, ladite cellule comprenant une cellule de levure hôte avec, dans le chromosome, ledit ADN d'origine de vecteur qui a été intégré ou un descendant de cette cellule de levure hôte dans laquelle ledit ADN d'origine de vecteur a été intégré, ledit ADN d'origine de vecteur comprenant tout d'abord un gène d'une protéine hétérologue souhaitée et ensuite une séquence d'ADN homologue à une séquence dudit chromosome dans ladite cellule de levure hôte.

12. Cellule selon l'une quelconque des revendications précédentes, caractérisée de plus en ce que ledit gène d'une protéine hétérologue souhaitée code pour la glucosamylase.

13. Cellule selon l'une quelconque des revendications 1 à 11 caractérisée de plus en ce que ledit gène d'une protéine hétérologue souhaitée code pour l'enzyme malolactique ou la malate-perméase.

14. Cellule selon la revendication 12, caractérisée de plus en ce que ledit vecteur permet l'expression de la glucosamylase dans ladite cellule de levure hôte.

15. Cellule selon les revendications 12 ou 14, caractérisée en ce que ladite glucosamylase permet à ladite cellule de produire du glucose à partir d'amidon.

16. Cellule selon la revendication 15, caractérisée de plus en ce que ladite cellule participe dans un procédé utilisant un produit du métabolisme de ladite cellule, ladite cellule utilisant ledit glucose en tant que source d'énergie dans ledit procédé.

17. Cellule selon la revendication 16, caractérisée de plus en ce que ledit produit du métabolisme est le dioxyde de carbone et ledit procédé est la production de pâte.

18. Cellule selon la revendication 16, caractérisée de plus en ce que ledit produit du métabolisme est l'éthanol.

19. Cellule selon la revendication 13, caractérisée de plus en ce que ledit ADN de vecteur permet l'expression de ladite enzyme malolactique ou ladite malate-perméase dans ladite cellule de levure hôte.

20. Cellule selon les revendications 13 ou 19, caractérisée en ce que ladite enzyme malolactique ou ladite malate-perméase permettent à ladite cellule de métaboliser l'acide malique.

21. Procédé de direction de l'intégration de l'ADN de vecteur dans le chromosome d'une cellule de levure non haploïde industrielle par l'utilisation d'un vecteur de clonage comprenant l'ADN de vecteur indiqué dans l'une quelconque des revendications 1 ou 6 à 9, et le procédé comprenant la linéarisation dudit vecteur dans ladite séquence homologue, puis l'exposition dudit vecteur linéarisé à ladite cellule de levure hôte dans des conditions de transformation.

22. Procédé de direction de l'intégration dans l'ADN chromosomique d'une souche de levure non haploïde industrielle telle que définie dans la revendication 6 d'un vecteur linéarisé tel que défini dans la revendication 6, au moins une portion de ladite région de non homologie partielle étant de ce fait enlevée dudit vecteur, ledit procédé comprenant :
l'exposition dudit vecteur linéarisé auxdites cellules de levure hôtes dans des conditions de transformation,
le choix, en tant que produits transformés, des cellules de levure présentant une résistance audit antibiotique et
l'isolement d'un sous-groupe desdits produits transformés qui ne présentent plus la résistance audit antibiotique.

23. Procédé de direction de l'intégration de l'ADN de vecteur dans l'ADN chromosomique d'une souche de levure non haploïde industrielle telle que définie dans l'une quelconque des revendications 7 à 9 par l'utilisation d'un vecteur linéarisé tel que défini dans l'une quelconque des revendications 7 à 9, respectivement, au moins une portion de ladite région de non homologie partielle étant de ce fait enlevée dudit vecteur, ledit procédé comprenant
l'exposition dudit vecteur linéarisé auxdites cellules de levure hôtes dans des conditions de transformation,
l'isolement, en tant que produits transformés, des cellules de levure présentant la résistance audit antibiotique et
l'isolement d'un sous-groupe desdits produits transformés ou descendants desdits produits transformés ne présentant pas ledit caractère pouvant être criblé.

24. Procédé selon la revendication 23, utilisant le vecteur défini dans la revendication 10, dans lequel l'isolement dudit sous-groupe est mis en oeuvre en faisant pousser lesdits produits transformés sur un milieu qui change de couleur en présence de β-galactosidase, puis en choisissant les produits transformés ne provoquant pas ledit changement de couleur.

25. Vecteur pRY253 (ATCC n° 39688).

26. Vecteur pRY255 (ATCC n° 39689).

27. Vecteur pRY255A (ATCC n° 39822).

28. Procédé de délétion d'un gène à partir du chromosome d'une cellule de levure non haploïde industrielle comprenant
la transformation de ladite cellule de levure avec un vecteur comprenant un gène de résistance à un antibiotique sinon capable de tuer ladite cellule de levure, ledit gène étant transcrit à partir d'une séquence promoteur qui est capable de favoriser l'expression dudit gène de résistance à l'antibiotique à un niveau qui confère la résistance à l'antibiotique à ladite cellule, ledit vecteur comprenant une séquence homologue à une séquence dudit chromosome et, de plus, l'ADN correspondant au gène dont il faut réaliser la délétion, ledit ADN comprenant une séquence d'ADN ayant des extrémités 3' et 5' homologues aux extrémités 3' et 5' dudit gène dont il faut réaliser la délétion et étant dénué du gène complet dont il faut réaliser la délétion et
l'isolement des produits transformés dénués dudit gène dont il faut réaliser la délétion.

29. Utilisation des cellules selon la revendication 11 pour produire de l'éthanol.

30. Utilisation des cellules selon la revendication 11 pour produire de la bière légère.

31. Utilisation des cellules selon la revendication 11 pour la production de pâte.

32. Utilisation des cellules selon la revendication 11 pour la panification de produits de type pain.

33. Utilisation des cellules selon la revendication 11 pour la fermentation de vin.
